(19) **Europäisches Patentamt European Patent Office Office européen des brevets**

(11) **EP 2 299 842 B1**

(12) **EUROPEAN PATENT SPECIFICATION**

(45) Date of publication and mention
of the grant of the patent:
**13.11.2013 Bulletin 2013/46**

(51) Int Cl.:
***A23K 1/16*** *(2006.01)*    ***A23K 1/18*** *(2006.01)*

(21) Application number: **09765768.8**

(22) Date of filing: **09.06.2009**

(86) International application number:
**PCT/EP2009/057069**

(87) International publication number:
**WO 2009/153188 (23.12.2009 Gazette 2009/52)**

(54) **USE OF 25-HYDROXYVITAMIN D3 FOR THE REDUCTION OF DRIP LOSSES IN MEAT**

VERWENDUNG VON 25-HYDROXYVITAMIN D3 ZUR VERRINGERUNG VON DRIPVERLUSTEN VOM FLEISCH

UTILISATION DE 25-HYDROXYVITAMINE D3 POUR LA RÉDUCTION DE PERTE DE JUS DE VIANDE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **19.06.2008 EP 08011134**

(43) Date of publication of application:
**30.03.2011 Bulletin 2011/13**

(73) Proprietor: **DSM IP Assets B.V.**
**6411 TE Heerlen (NL)**

(72) Inventors:
• **PINON-QUINTANA, Arturo**
**F-68100 Mulhouse (FR)**
• **SIMOES-NUNES, Carlos**
**F-68128 Village-Neuf (FR)**

(74) Representative: **Schiener, Jens et al**
**Lederer & Keller**
**Patentanwälte**
**Unsöldstrasse 2**
**80538 München (DE)**

(56) References cited:
EP-A- 1 516 540    WO-A-99/04648
WO-A-03/059358    US-A1- 2002 198 185

• WILBORN BS ET AL: "Improving pork quality by feeding supranutritional concentrations of vitamin D3" J. ANIM. SCI., vol. 82, 2004, pages 218-224, XP002543858
• KARGES K ET AL: "Effects of supplemental vitamin D3 on feed intake, carcass characteristics, tenderness, and muscle properties of beef steers" J. ANIM. SCI., vol. 79, 2001, pages 2844-2850, XP002543859
• WIEGAND BR ET AL: "Short-term feeding of vitamin D3 improves color but does not change tenderness of pork-loin chops" J. ANIM. SCI., vol. 80, 2002, pages 2116-2121, XP002543860
• RIDER SELL N, MIKEL WB, XIONG YL, BEHRENDS JM: "Vitamin D3 supplementation of cull cows: effects on longissimus and semitendinosus muscle tenderness" J. ANIM. SCI., vol. 82, 2004, pages 225-230, XP002543861
• Anonymous: "Opinion of the scientific panel on additives and products or substances used in animal feed on a request from the Commission on the evaluation of safety and efficacy of "Hy-D" (calcifediol), based on 25-hydroxycholecalciferl/25-hydroxy-pre-ch olecalciferol, as feed additive...", The EFSA Journal, vol. 224 2005, pages 1-35, Retrieved from the Internet: URL:http://www.iss.it/binary/sani/cont/cal cifediolo.1121428025.pdf [retrieved on 2011-07-25]

EP 2 299 842 B1

## Description

BRIEF DESCRIPTION OF THE INVENTION

This invention relates to uses of 25-hydroxyvitamin D3, in particular for reducing drip losses in pork comprising administering to 25-hydroxyvitamin D3 to a pig. This invention also relates to uses of 25-hydroxyvitamin D3 in swine feeds and premixes which are effective in reducing drip losses.

BACKGROUND OF THE INVENTION

Water holding capacity (WHC) and color are major quality attributes of fresh pork. They affect the appearance of the fresh meat and thus the consumer's perception at the time of purchase. Moreover, WHC affects both the technological value of the fresh meat and the juiciness of the cooked or cured product.

In the past, many factors have been investigated as involved in meat quality. Some genes have been identifies as contributing to unacceptable carcass quality. For example, the presence of the halothane gene resulted in high frequency of PSE (pale, soft and exsudative) pork carcasses. However, successful breeding strategies have now more or less eliminated halothane carriers in the European pig population with the subsequent elimination of extreme PSE cases. However, the elimination of this gene has not totally solved entirely the problem, as differences in WHC and pale color are still present.

It was found in the USA and in some European countries that important economic losses are associated with quality defects which differ from typical PSE meat, such as RSE (red, soft and exsudative) meat. In any case the poor WHC of the meat is a fundamental aspect of both defects.

The influence of stress and of nutrition on the meat quality of pigs has been extensively studied. Several factors have been considered as having an influence on the WHC of the pork meat. Among the nutritional factors one should also consider vitamins and trace elements. Thus, it would be desirable to be able to reduce drip losses in pork meat by influencing the diet of the growing-fattening pigs. 25-Hydroxyvitamin D3 has been added to porcine diets in the past. See, for example WO03/059358, WO05/664018, and EP1516540. However, none of these applications teach the ability of 25-hydroxyvitamin D3 to reduce the amount of drip loss.

Wilborn et al., J. Anim. Sci., Vol. 82, 2004, 218-224, disclose improving pork quality by feeding supranutritional concentrations of vitamin D3.

Karges et al., J. Anim. Sci., Vol. 79, 2001, 2844-2850, disclose effects of supplemental vitamin D3 on feed intake, carcass characteristics, tenderness, and muscle properties of beef steers.

"Opinion of the scientific panel on additives and products or substances used in an animal feed on a request from the Commission on the evaluation of safety and efficacy of "Hy-D" (calcifediol), based on 25-hydroxycholecalciferol/ 25-hydroxy-pre-cholecalciferol, as feed additive in accordance with Council Directive 70/524/EEC", The EFSA Journal, Vol. 224, 2005, 1-35 discloses the use of 25-OH D3 for chickens and turkeys.

It would be desirable to be able to manage the amount of drip-loss in fresh meat through dietary means.

DETAILED DESCRIPTION OF THE INVENTION

It has been found that by feeding an animal feed supplemented with 25-hydroxyvitamin D3, the water holding capacity (WHC) of the resulting meat can be increased, and thus drip loss of the carcass can be reduced. Thus, the present invention relates to the subject matter of claims 1 to 8. One aspect of this invention is using an animal feed supplemented with 25-hydroxyvitamin D3 in an amount sufficient to improve WHC of the resulting meat.

In particular, it has been found, in accordance with this invention that the carcasses of growing-fattening pigs which received 25-hydroxycholecalciferol (also called 25-hydroxy vitamin D3; 25-OH D3) in the diet had a reduced amount of drip losses. Thus, in one aspect of this invention 25-hydroxyvitamin D3 is used to reduce drip losses in pork meat to growing/fattening pigs.

Yet another aspect of this invention is using 25-OH D3 in a pre-mix.

25-OH Vitamin D3 diet can be used to prevent drip loss in any animal which may exhibit exsudative meat. Preferably the animal is a pig or calf.

Dosages:

The dosage which is effective will, of course, depend on the species of animal which is being reared. For swine, about 40 to 60 $\mu$g/kg of 25-hydroxyvitamin D3 and preferably 50 $\mu$g/kg of 25-hydroxyvitamin D3 is effective. This dosage is preferably given during all the growing-fattening phase of pigs for fattening (i.e. from about 60 days onward). In some embodiments, this dosage is constant during the entire growing-fattening phase.

The 25-OH D3 is preferably part of a vitamin pre-mix at a convenient concentration which can easily be mixed in to provide the above dosages. The concentrated vitamin pre-mix can be mixed with feed, wherein the amount of 25-OH Vitamin D3 in the finished feed is from 25-75 $\mu$g/kg, preferably 40-60 $\mu$g/kg of 25-hydroxyvitamin D3 and even more preferably about 50 $\mu$g/kg of 25-hydroxyvitamin D3. The finished feed contains 25-75 $\mu$g/kg, preferably 40-60 $\mu$g/kg of 25-hydroxyvitamin D3 and preferably about 50 $\mu$g/kg of 25-hydroxyvitamin D3.

The following non-limiting Examples are presented to further illustrate the invention.

## EXAMPLES

## Example 1

## Evaluation of the effects of the long term dietary supplementation with 25-hydroxycholecalciferol on the carcass yield, lean/fat ratio and drip loses of the growing-fattening pig.

Fifty 28-d old Large-White x Landrace weaner piglets having an initial body weight of 7.9$\pm$0.74 kg were used. The animals were allocated into two equal groups (A and B) and housed in cages in sub-groups (two of 8 and one of 9 animals) in an environmentally controlled room. Each cage had a plastic-coated welded wire floor and was equipped with 2 water nipples and 2 stainless-steel feeders. Room temperature was initially 27°C and was lowered weekly by about 2°C until 21-22°C. Environment humidity percentage throughout the experiment was 50 %. The experiments were performed in the facilities of the Centre de Recherche en Nutrition Animale (CRNA), DSM Nutritional Products France, BP 170, 68305 Saint-Louis cedex, France. They have been performed according to the French legal regulations on experiments with live animals.

Each group of animals was fed either a basal diet with the addition of 2000 IU/kg of vitamin $D_3$ (group A) or the basal diet, without vitamin $D_3$, and supplemented with 50 $\mu$g/kg of 25-hydroxycholecalciferol (Rovimix® Hy•D® commercially available from DSM Nutritional Products) (group B).

Both diets were distributed ad libitum in a mash form. The basal diet was formulated to meet the animals' requirements according to Henry et al. (1989) and NRC (1998).

The composition of the basal diet and the concentrations of vitamin $D_3$ and of 25-OH D3 in the supplemented diets are presented in Table 1.

**Table 1. Composition of the experimental diets. Post-weaning phase.**

| Ingredients (%) | | A | B |
|---|---|---|---|
| Soybean meal | | 7.4 | |
| Wheat | | 15 | |
| Barley | | 29.4 | |
| Potato concentrate | | 8 | |
| Maize | | 10 | |
| Wheat bran | | 1.6 | |
| Oatmeal | | 10 | |
| Beet pulp | | 5.5 | |
| Treacle | | 3.5 | |
| Soya oil | | 2.7 | |
| Minerals, vitamins and synthetic amino acids | | 6.9 | |
| Crude protein - N x 6.25 - % | | 17.9 | |
| Lysine - % | | 1.3 | |
| Methionine + cystine - % | | 0.8 | |
| Estimated digestible energy - MJ/kg | | 13.8 | |
| Ca - % | | 0.8 | |
| P - % | | 0.6 | |
| Vitamin $D_3$ (IU/kg) | target level | 2000 | - |
| | analyzed content | 1900[(1)] | - |

(continued)

| Ingredients (%) | | A | B |
|---|---|---|---|
| 25-OH D3 ($\mu$g/kg) | target level | - | 50 |
| | analyzed content | - | 57.1[1] |
| [1] - Mean of 2 determinations. | | | |

At the end of the post-weaning phase the twenty heaviest animals of each group were used for the growing-fattening period. They had an initial body weight of 19.4±1.36 kg for the group A and of 21.5±1.46 kg for the group B. The animals were housed in floor-pen cages in sub-groups of 4 animals each in an environmentally controlled room. Each pen had a plastic-coated welded wire floor and was equipped with two water nipples and four stainless-steel individualized feeders. Room temperature was 21-22° C and humidity percentage was 50 %.

The pigs were fed for 87 days a diet supplemented either with 2000 IU/kg of vitamin $D_3$ (group A, animals ingesting during the post-weaning phase 2000 U/kg) or with 50 $\mu$g/kg of 25-hydroxycholecalciferol (group B, animals ingesting during the post-weaning phase 50 $\mu$g/kg). The basal diet (Table 2) was formulated to meet the animals' requirements according to Henry et al. 1989 In: L'alimentation des animaux domestiques - porc, lapin, volailles (ed. INRA), 2ème edition, INRA, Paris, 49-76 and NRC, 1998. Nutrient requirements of swine, 10th revised edition, National Academic Press. Washington DC, U.S.A.

**Table 2. Composition of the experimental diets. Growing-fattening phase.**

| Ingredients (%) | | A | B |
|---|---|---|---|
| Maize | | 53 | |
| Soybean meal | | 18.2 | |
| Barley | | 13 | |
| Oat meal | | 6 | |
| Wheat bran | | 5.4 | |
| Soya oil | | 1 | |
| Minerals, vitamins and synthetic amino acids | | 3.4 | |
| Crude protein - N x 6.25 - % | | 15.5 | |
| Lysine - % | | 0.96 | |
| Methionine + cystine - % | | 0.54 | |
| Estimated digestible energy - MJ/kg | | 13.31 | |
| Ca - % | | 0.66 | |
| P - % | | 0.41 | |
| Vitamin $D_3$ (IU/kg) | target level | 2000 | - |
| | analyzed content | 2350[1] | - |
| Hy·D® ($\mu$g/kg) | target level | - | 50 |
| | analyzed content | - | 54±10.6[2] |
| [1] - Mean of 2 determinations; [2] - Mean ± standard deviation of 3 mixing protocols. | | | |

The health status of the animals has been controlled daily and specific observations (in stand and walking attitudes) were done during animals' weighing for detection of limb or foot problems.

At the end of the experiment, all animals were randomly divided into four different lots and assigned to one slaughter campaign: days 84th, 85th, 86th and 87th. Animals of each lot were fasted for 12 hours and weighed just before they were slaughtered after tranquilization and stunning. The carcasses were sawed longitudinally following the spinal canal and weighed after 24 hours storage at +4°C to estimate the carcasses' cold yield.

In one of the mid-carcass, the entire ilio-spinalis muscle (*longissimus dorsi*) was dissected. Each muscle was weighed and pH was measured immediately and after 24 hours storage at +4°C. The muscles were individually conditioned into inflated plastic bags for water recovery during storage. Percentage of drip loses from muscles was estimated by weight difference and water recovery.

# EP 2 299 842 B1

In the second mid-carcass lean and fat were measured at three different points with a caliper square tool. Muscular depth was measured after dissection of the ilio-spinalis muscle (*longissimus dorsi*) at the level of the $4^{th}$ dorsal vertebrae. The sub-cutaneous back fat deposition was measured at the levels of the $4^{th}$ dorsal and lumbar vertebras. These measures were used to estimate the carcasses' lean/fat ratio.

<u>Statistical analysis</u>

Statistical treatment of the results involved the calculation of the mean and the standard deviation of the mean as well as a two-factor hierarchical analysis of variance at 95% confidence level. The mathematical model was:

$$Yijk = \mu + Ai + Bij + Zijk$$

where $\mu$ is the mean, Ai is the diet effect, Bij is the combined effect of the diet and animal or pen and Zijk is the residual term. The analysis of variance was followed by a Student test when a significant Ai effect without Bij effect was observed (Snedecor and Cochran, 1989 Statistical methods, 8th edition, Iowa University Press, Ames). These calculations were performed using StatGraphics Plus 5.1 (Manugistics, Rockville, U.S.A. 2001). All parameters were analyzed using individuals as experimental units.

<u>Results</u>

The observed vitamin $D_3$ and 25-hydroxycalciferol concentration in the supplemented feed used was in good agreement with the programmed inclusion levels (Tables 1 and 2). The animals did not present symptoms of illness during any phase of the experiment and no limb or foot problems were detected. The dietary 25-OH D3 supplementation had no positive effects on the cold yield and the lean/fat ratio of the carcasses as can be seen in the Tables 3 and 4, below.

**Table 3. Effects of the addition to the diet of 25-OH D3 on the carcasses' cold yield (% of live weight).**

| A<br>2000 U/kg $D_3$ | B<br>50 $\mu$g/kg 25-OH D3 |
|---|---|
| $81.85 \pm 0.76^{(1)}$ (100) | $81.11 \pm 1.71$ (99) |
| Diet based on: maize, barley and soybean meal;<br>Animals: pigs of an initial body weight of $19.4 \pm 1.36$ kg (group A) and of $21.5 \pm 1.46$ kg (group B);<br>$^{(1)}$ - mean $\pm$ standard deviation of the mean of 20 determinations. | |

**Table 4. Effects of the addition to the diet of Hy•D® on the carcasses' lean / fat ratio (%).**

| A<br>2000 U/kg $D_3$ | B<br>50 $\mu$g/kg 25-OH D3 |
|---|---|
| $68.42 \pm 2.26^{(1)}$ (100) | $67.70 \pm 2.99$ (99) |
| Diet based on: maize, barley and soybean meal;<br>Animals: pigs of an initial body weight of $19.4 \pm 1.36$ kg (group A) and of $21.5 \pm 1.46$ kg (group B);<br>$^{(1)}$ - mean $\pm$ standard deviation of the mean of 20 determinations. | |

The measured pH values immediately after slaughter and after 24 hours storage. Ilio-spinalis muscle segments were similar in animals ingesting 25-OH D3 when compared to values from animals of the control group (Table 5).

**Table 5. Effects of the addition to the diet of 25-OH D3 on the pH and the drip losses (%) of the ilio-spinalis (*longissimus dorsi*) muscle.**

| Variables | | A<br>2000 U/kg $D_3$ | B<br>50 $\mu$g/kg 25-OH D3 |
|---|---|---|---|
| pH | At slaughter | $5.85 \pm 0.21^{(1)}$ (100) | $5.89 \pm 0.16$ (101) |
| | after 24 h | $5.49 \pm 0.05^{(1)}$ (100) | $5.51 \pm 0.04$ (100) |

(continued)

| Variables | | A<br>2000 U/kg $D_3$ | B<br>50 µg/kg 25-OH D3 |
|---|---|---|---|
| Drip loss (%) | by weight difference | 1.94±0.85[1] (100) | 1.51±0.28 (78) |
| | by water recovery | 0.78±0.74[1] (100) | 0.45±0.23 (58) |
| Diet based on: maize, barley and soybean meal;<br>Animals: pigs of an initial body weight of 19.4±1.36 kg (group A) and of 21.5±1.46 kg (group B);<br>[1] - mean ± standard deviation of the mean of 20 determinations. | | | |

Intracellular acidification of myocytes is one of the three main factors, together with conformation of actin-myosin complex (*rigor mortis*) and protein denaturalization, which modifies myofibrilar volumes (Monin, 2003 INRA. Prod. Anim. 16(4), 251). After slaughter, intracellular pH falls from 7 to about 5.4 - 5.7, inducing myofibrilar contraction and reducing the available space for water retention within the muscular cells.

Drip losses and pH values measured in this study are in agreement with lower pH values and higher drip losses observed by Alarcón Rojo *et al.* 2006 Alarcón Rojo A. D. et al. 2006. Tec Pecu Méx. 44 (1):53 in carcasses of pigs submitted to different stressing factors when compared to those from animals undergoing optimal process conditions during slaughter.

It has been reported that rapid $Ca^{2+}$-triggered endomembrane exocytosis is a crucial step in the membrane resealing process and that 1,25-dyhydroxycholecalciferol stimulated elevation of intracellular calcium due to the activation of $Ca^{2+}$ channels in osteoblasts.

Without wishing to be bound by theory, it appears that dietary supplementation of 25-OH D3 may, indirectly enable the repairing capacity of muscular cells in response to cell membrane disruptions as consequence of *port mortem* processes. Once water reaches extra-cellular spaces it can flow through muscular fibers dropping out at muscle edges. In the present experiment, clearly smaller water volumes were recovered and less mass was lost from muscular segments of pigs receiving 25-OH D3 (Table 5).

## EXAMPLE 2

A pig food containing 25-hydroxyvitamin D3 can be prepared as follows:

| Ingredients | [%] |
|---|---|
| Soybean meal | - 18 |
| Maize | - 53 |
| Barley | - 14 |
| Oat meal | - 6 |
| Wheat bran | - 5.4 |
| Soy oil | - 1 |
| Minerals | - 1.5 |
| Synthetic amino acids premix | - 0.5 |
| Vitamins and trace elements premix | - 0.6 |

(containing from about 0.03 to 0.099g of Hy·D® 1.25% Beadlet per 100 g of premix) The ingredients are mixed together and if needed the obtained mash food can be pelleted.

## EXAMPLE 3

A premix for a pig food containing 25-hydroxyvitamin D3 can be prepared as follows:

| Ingredients | [%] |
|---|---|
| Hy·D® 1.25% Beadlet | 0.08 |
| Vitamin A 500 | 0.8000 |
| Vitamin E 50% | 8.0000 |
| Vitamin K3 100% MSB / 51% | 0.0800 |

(continued)

| Ingredients | [%] |
|---|---|
| Vitamin B1 98% | 0.0714 |
| Vitamin B2 80% | 0.1750 |
| Vitamin B6 99% | 0.1212 |
| Vitamin B12 0.1% | 1.0000 |
| Biotin 2% | 0.2000 |
| Folic Acid 80% | 0.0227 |
| Niacin 99.5% | 0.7035 |
| Calpan 98% | 0.4082 |
| Vitamin C | 4.0000 |
| Cholinchloride 60% | 12.0000 |
| Copper sulfate 25% | 12.8000 |
| Iron sulfate 30% | 10.0000 |
| Mangan oxide 62% | 1.6129 |
| Zinc oxide 76% | 5.2632 |
| Cobalt carbonate 5% | 0.0600 |
| Calcium jodate 62% | 0.0323 |
| Sodium selenite 1%/BMP | 0.8001 |
| BHT 100% | 2.0000 |
| Carrier Combination | 6.0000 |
| LACANTES S36400-Z | 10.0000 |
| Limestone | 23.7375 |

All ingredients are carefully mixed together and 0.5% (5kg/1000 kg of food) of this premix is added to the final pig food. With the addition of such premix the 25-hydroxyvitamin D3 concentration in the prepard feed will be 50µg/kg.

Alternatively, 25-hydroxyvitamin D3 can also be added in a 1% diluted premix, containing a suitable carrier. Such carrier can be wheat flour, wheat middlings, corn cobs, rice hulls, almond shells or calcium carbonate alone or in variable mixtures of several of these carriers. A typical formula is:

| Ingredients | [%] |
|---|---|
| Wheat flour | 80.00 |
| Calcium carbonate | 19.2 |
| Hy·D® 1.25% Beadlet | 0.8 |

All ingredients are carefully mixed together and 0.05% (0.5 kg/1000 kg of food) of this premix is added to the final pig food. With the addition of such premix, the 25-hydroxyvitamin D3 concentration in the prepared feed will be 50µg/kg.

**Claims**

1. Use of 25-hydroxyvitamin D3 for increasing the water holding capacity (WHC) of meat obtained from an animal ingesting a supplemented feed comprising 25 to 75 µg/kg, preferably 40 to 60 µg/kg of 25-hydroxyvitamin D3 and more preferably about 50 µg/kg of 25-hydroxyvitamin D3.

2. Use according to claim 1 wherein the animal is swine or calf.

3. Use according to claims 1 or 2 wherein the feed is for growing/fattening pigs.

4. Use according to any of claims 1 to 3 wherein the 25-hydroxyvitamin D3 is present in a feed pre-mix.

5. Use of 25-hydroxyvitamin D3 for reducing drip losses of meat obtained from an animal ingesting a supplemented feed comprising 25 to 75 µg/kg, preferably 40 to 60 µg/kg of 25-hydroxyvitamin D3 and more preferably about 50

$\mu$g/kg of 25-hydroxyvitamin D3.

**6.** Use according to claim 5, wherein the animal is swine.

**7.** Use according to claim 5 or 6, wherein the feed is for growing/fattening pigs.

**8.** Use according to claims 5 to 7, wherein the 25-hydroxyvitamin D3 is present in a feed pre-mix.

**Patentansprüche**

**1.** Verwendung von 25-Hydroxy-Vitamin-D3 zur Erhöhung des Wasserhaltevermögens (WHC) von Fleisch, erhalten von einem Tier, das ergänztes Futtermittel zu sich nimmt, das 25 bis 75 $\mu$g/kg, bevorzugt 40 bis 60 $\mu$g/kg 25-Hydroxy-Vitamin-D3 und stärker bevorzugt etwa 50 $\mu$g/kg 25-Hydroxy-Vitamin-D3 umfasst.

**2.** Verwendung nach Anspruch 1, wobei das Tier ein Schwein oder ein Kalb ist.

**3.** Verwendung nach Anspruch 1 oder 2, wobei das Futtermittel für das Heranziehen/Mästen von Schweinen vorgesehen ist.

**4.** Verwendung nach einem der Ansprüche 1 bis 3, wobei das 25-Hydroxy-Vitamin-D3 in einer Futtermittelvormischung vorliegt.

**5.** Verwendung von 25-Hydroxy-Vitamin-D3 zur Reduktion von Tropfverlusten von Fleisch, erhalten von einem Tier, das ergänztes Futtermittel zu sich nimmt, das 25 bis 75 $\mu$g/kg, bevorzugt 40 bis 60 $\mu$g/kg 25-Hydroxy-Vitamin-D3 und stärker bevorzugt etwa 50 $\mu$g/kg 25-Hydroxy-Vitamin-D3 umfasst.

**6.** Verwendung nach Anspruch 5, wobei das Tier ein Schwein ist.

**7.** Verwendung nach Anspruch 5 oder 6, wobei das Futtermittel zum Heranziehen/Mästen von Schweinen vorgesehen ist.

**8.** Verwendung nach den Ansprüchen 5 bis 7, wobei das 25-Hydroxy-Vitamin-D3 in einer Futtermittelvormischung vorliegt.

**Revendications**

**1.** Utilisation de 25-hydroxyvitamine D3 pour accroître la capacité de rétention d'eau de la viande obtenue à partir d'un animal ingérant une alimentation enrichie comprenant 25 à 75 $\mu$g/kg, de préférence 40 à 60 $\mu$g/kg de 25-hydroxyvitamine D3 et mieux encore environ 50 $\mu$g/kg de 25-hydroxyvitamine D3.

**2.** Utilisation selon la revendication 1, l'animal étant un porc ou un veau.

**3.** Utilisation selon les revendications 1 ou 2, l'aliment servant à élever/engraisser des cochons.

**4.** Utilisation selon l'une quelconque des revendications 1 à 3, la 25-hydroxyvitamine D3 étant présente dans un pré-mélange pour l'alimentation des animaux.

**5.** Utilisation de 25-hydroxyvitamine D3 pour réduire les pertes de liquide de la viande obtenue à partir d'un animal ingérant une alimentation enrichie comprenant 25 à 75 $\mu$g/kg, de préférence 40 à 60 $\mu$g/kg de 25-hydroxyvitamine D3 et mieux encore environ 50 $\mu$g/kg de 25-hydroxyvitamine D3.

**6.** Utilisation selon la revendication 5, l'animal étant un porc.

**7.** Utilisation selon la revendication 5 ou 6, l'aliment servant à élever/engraisser des cochons.

**8.** Utilisation selon les revendications 5 à 7, la 25-hydroxyvitamine D3 étant présente dans un pré-mélange pour l'alimentation des animaux.

**EP 2 299 842 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03059358 A
- WO 05664018 A
- EP 1516540 A

**Non-patent literature cited in the description**

- **WILBORN et al.** *J. Anim. Sci.,* 2004, vol. 82, 218-224
- **KARGES et al.** *J. Anim. Sci.,* 2001, vol. 79, 2844-2850
- *The EFSA Journal,* 2005, vol. 224, 1-35
- **HENRY et al.** L'alimentation des animaux domestiques - porc, lapin, volailles. INRA, 1989, 49-76
- Nutrient requirements of swine. revised edition, National Academic Press, 1998
- **SNEDECOR ; COCHRAN.** Statistical methods. Iowa University Press, 1989
- **MONIN.** *INRA. Prod. Anim.,* 2003, vol. 16 (4), 251
- **ALARCÓN ROJO A. D. et al.** *Tec Pecu Méx.,* 2006, vol. 44 (1), 53